# EUROPEAN PATENT SPECIFICATION

(11) **EP 1 997 529 B1**
(45) Date of publication and mention of the grant of the patent: **13.05.2015**
(21) Application number: 08251920.8
(22) Date of filing: 02.06.2008
(51) Int. Cl.: A61N 1/36, A61N 1/362

(54) **System for the treatment of cardiomypathy, heart failure and cardiac ischemia using stimulation induced secretion of GLP-1**
System zur Behandlung von Kardiomyopathie, Herzversagen und Herzischämie mittels induzierter GLP-1-Sekretion durch Stimulation
Dispositif pour le traitement de myocardiopathie, insuffisance cardiaque et ischémie cardiaque utilisant le sécrétion de GLP-1 induite par stimulation

(30) Priority: 31.05.2007 US 756478
(43) Date of publication of application: 03.12.2008
(73) Proprietor: PACESETTER, INC., Sylmar, CA 91392-9221 (US)
(72) Inventor: Cholette, Martin, Acton, CA 93510 (US)
(74) Representative: Noble, Nicholas

(56) References cited:
- EP-A- 1 486 232
- WO-A-2004/112563
- US-A1- 2005 043 675
- US-A1- 2007 060 971
- ROCCA A S ET AL: "Role of the vagus nerve in mediating proximal nutrient-induced glucagon-like peptide-1 secretion." ENDOCRINOLOGY APR 1999, vol. 140, no. 4, April 1999 (1999-04), pages 1687-1694, XP002490653 ISSN: 0013-7227

## Description

Exemplary methods, devices, systems, etc., presented herein generally relate to implantable devices for delivery of cardiac therapy.

According to epidemiologic data, as many as 5 million Americans are afflicted with heart failure (HF). For many of these patients, health related quality of life (HRQoL) is poor and the 5 year mortality rate is extremely high. Further, the rate of hospitalizations for HF increased more than three times between 1970 and 1994 at age 45 to 64 and at age 65 and older. With the use of angiotensin-converting enzyme (ACE) inhibitors and beta blockers as possible exceptions, advances in drug therapy for hypertension, myocardial ischemia, and valvular heart disease have not resulted in substantial improvements in survival once congestive heart failure (CHF) ensues. Non-drug therapies for HF include cardiac transplantation, mechanical pumps for ventricular assist and cardiac pacing. Such non-drug therapies can improve HRQoL and decrease mortality rate.

As many HF patients have health conditions that can affect cardiac performance, an understanding of comorbidity can help in selecting appropriate treatment. In general, the term comorbidity is used to indicate a medical condition in a patient that causes, is caused by, or is otherwise related to another condition in the same patient. For example, a primary condition of interest may be HF and diabetes may be a related, secondary condition. Indeed, various studies show that CHF incidence rate in type 2 diabetes patients, and hence comorbidity, is significant and that treatment should focus on controlling modifiable risk factors for CHF, namely hyperglycemia, elevated blood pressure, and obesity.

Type 2 diabetes is a metabolic disorder that is epidemic in the United States and around the world. More than 150 million people in the world are diabetic and, by the year 2010, over 200 million people are expected to be diabetic. In the U.S., for those born in the year 2000, estimated lifetime risk for diabetes is greater than 1 in 3. The economic and human cost of this disease is devastating. The current cost of diabetes in the U.S. is estimated to be at $132 billion, which includes $92 billion of direct medical costs and $40 billion indirect costs such as disability, work loss and premature mortality. The current diabetic epidemic has been accompanies by a similar increase in obesity. Traditional glucophage medications do not offer an acceptable treatment for most type 2 diabetics and many progress to develop life shortening and debilitating comorbidities such as hypertension, cardiovascular disease, renal disease, neuropathies, blindness, etc.

Various exemplary devices, systems, methods, etc., discussed herein aim to treat HF using techniques that directly promote cardiac health or that indirectly promote cardiac health by treating secondary conditions (e.g., metabolic disorders) that have been shown to be related to HF.

US-A-20050043675 discloses a drug delivery system arranged to delivery a drug when an ischemic condition is detected.

The invention is set out in the claims. According to the invention there is provided an implantable device for diminishing long-term myocardial damage caused by an infarct or ischemia, the device comprising, detection circuitry adapted to detect an infarct or ischemia, and stimulation circuitry adapted to call, in response to the detection circuitry detecting an infarct or ischemia, for delivery of electrical stimulation to a vagal pathway and/or enteric nerve pathway to activate L cells in the distal ileum wherein activation of L cells causes release of GLP-1 and wherein release of GLP-1 diminishes long-term myocardial damage caused by an infarct or ischemia.

An exemplary method for treating heart failure includes diagnosing heart failure and calling for delivery of energy to the distal ileum to activate L cells using an implantable device where activation of L cells causes release of GLP-1 and where release of GLP-1 treats heart failure. Various other methods, devices, systems, etc., are also disclosed.

An exemplary method for diminishing long-term myocardial damage caused by an infarct or ischemia comprises: detecting an infarct or ischemia; and calling for delivery of energy to the distal ileum to activate L cells using an implantable device wherein activation of L cells causes release of GLP-1 and wherein release of GLP-1 diminishes long-term myocardial damage caused by an infarct or ischemia. The method may further comprise calling for delivery of energy to the duodenum to activate K cells using an implantable device wherein activation of K cells causes release of GIP, and may comprise calling for coordinated delivery of energy to the duodenum and to the distal ileum.
The detecting an infarct or ischemia may comprise acquiring an electrogram and the method may comprise calling for delivery of energy to the distal ileum to activate L cells in response to acquiring an electrogram that indicates occurrence of an infarct or ischemia. The detecting an infarct or ischemia may comprise acquiring at least one temperature or at least one temperature differential and the method may comprise calling for delivery of energy to the distal ileum to activate L cells in response to detecting an abnormal temperature or an abnormal temperature differential. The temperature or the temperature differential may comprise a cardiac temperature or a cardiac temperature differential.
The method may comprise calling for delivery of energy to the distal ileum to activate L cells in response to detecting a low concentration of GLP-1. The concentration of GLP-1 may comprise a gastrointestinal concentration or a blood concentration. The method may comprise calling for delivery of energy to the distal ileum to activate L cells in response to detecting a high in vivo concentration of glucose. The concentration of glucose may comprise a gastrointestinal concentration or a blood concentration.

A further exemplary method for diminishing long-term myocardial damage caused by an infarct or ischemia comprises: detecting an infarct or ischemia; and activating at least one member selected from a group consisting of vagal nerves, K cells and L cells by using an implantable device, wherein the activating induces, directly or indirectly, release of GLP-1 diminishes long-term myocardial damage caused by an infarct or ischemia.

Features and advantages of the described implementations can be more readily understood by reference to the following description taken in conjunction with the accompanying drawings.

Fig. 1 is a diagram illustrating various exemplary scenarios where an implantable device interacts with proximal site(s) and/or distal site(s) of the gastrointestinal tract in the body.

Fig. 2 is a functional block diagram of an exemplary implantable stimulation device illustrating basic elements that are configured to provide one or more therapies to treat, for example, a cardiac condition.

Fig. 3 is a series of block diagrams for various exemplary methods to induce release of GLP-1.

Fig. 4 is an approximate anatomical diagram that includes the stomach, the duodenum and the intestines.

Fig. 5 is a diagram of various mechanisms associated with release of GLP-1.

Figs. 6A and 6B are a series of block diagrams for various exemplary methods to directly or indirectly induce release of GLP-1.

Fig. 7 is an anatomical diagram that includes a portion of the duodenum and electrodes positioned subserosally.

Fig. 8 is an anatomical diagram that includes a portion of the ileum and the colon and electrodes positioned subserosally.

Fig. 9 is a diagram of an exemplary scheme for delivery of electrical energy to a proximal portion of the intestines and/or a distal portion of the intestines.

Fig. 10 is a block diagram of an exemplary method for treating a cardiac condition by a GLP-1 related mechanism.

Fig. 11 is a block diagram of an exemplary method for increasing GLP-1 concentration.

### Detailed Description

The following description includes the best mode presently contemplated for practicing the described implementations. This description is not to be taken in a limiting sense, but rather is made merely for the purpose of describing the general principles of the implementations. The scope of the described implementations should be ascertained with reference to the issued claims.

### Overview

Various exemplary therapies described herein treat HF by inducing secretion of GLP-1. Various studies using animal HF models show that administration of the entero hormone glucagon-like peptide 1 (GLP-1) can increase cardiac output and improve overall hemodynamics. Further, a study by Rocca and Brubaker shows that GLP-1 secretion can be induced in rats by using a Grass stimulator (Grass Instruments, Quincey, MA) to deliver electrical stimulation to the distal portion of the celiac branch of the vagal nerve ("Role of the Vagus Nerve in Mediating Proximal Nutrient-Induced Glucagon-Like Peptide-1 Secretion", Endocrinology 140: 1687-1694, 1999). Various therapies discussed herein use an implantable device to induce secretion of GLP-1 to treat a HF patient.

Described below are various exemplary system scenarios that include at least one implantable device, an example of circuitry that may be included in an implantable device, various exemplary methods for inducing secretion of GLP-1, various mechanisms for inducing secretion of GLP-1, approximate anatomical diagrams for the duodenum and distal ileum, some control logic schemes for use in delivering a therapy and some flow diagrams of some more specific exemplary methods.

Various exemplary devices, systems, methods, etc., call for delivery of electrical energy to one or more sites to control, directly and/or indirectly, one or more hormones that play a role in cardiac health. For example, an exemplary technique includes delivering electrical energy to a proximal site (e.g., at or near the duodenum, proximal segment of the jejunum, etc.) and a distal site (e.g., distal ileum near the cecum) to control release of one or more hormones. Such delivery of energy may activate and/or block activity (e.g., inhibit nutrient activation of a neuroendocrine mechanism). In addition to having an effect on cardiac health, such techniques may aid glycemic control, decrease insulin resistance, improve beta cell function (e.g., hormone synthesis and/or release), promote satiety, etc.

In addition, various studies indicate that autonomic mechanisms play a role in hormone synthesis, release and/or regulation. In particular, afferent and/or efferent parasympathetic pathways play a role. Various exemplary techniques may include activation and/or blockade of autonomic traffic. Such techniques may deliver electrical energy to the celiac branch of the vagus nerve.

An exemplary therapy may be substituted for a conventional therapy or used as an adjunct therapy for increasing effectiveness of a conventional therapy. An exemplary therapy may be delivered using open-loop or closed-loop techniques.

### Exemplary Scenarios

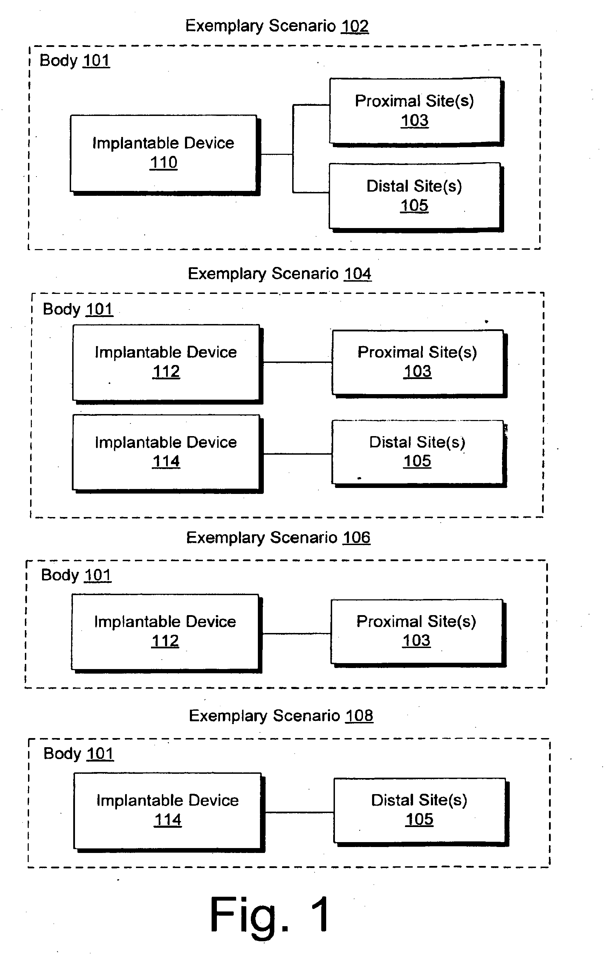

Fig. 1 shows various exemplary scenarios 102, 104 and 106 where at least one implantable device (e.g., 110, 112, 114, 118) interacts with a proximal gastrointestinal site 103 of the body 101 and/or interacts with a distal gastrointestinal site 105 of the body 101. In general, such interactions include interactions that activate and/or block activity associated with the gastrointestinal system to thereby help improve cardiac condition or cardiac performance. An exemplary implantable device may optionally provide for cardiac stimulation therapy or communicate with a device that can provide for a cardiac therapy (e.g., pacing therapy, defibrillation therapy, autonomic stimulation therapy, etc.). An exemplary implantable device may optionally provide for respiratory therapy or communicate with a device that can provide for respiratory therapy (e.g., apnea therapy, etc.). An implantable device optionally includes a drug reservoir and a pump to deliver a drug from the reservoir to a patient. Such a device may include a port for insertion of a needle to refill the reservoir.

The exemplary scenario 102 includes a single implantable device 110 that can sense, activate and/or block activity of a proximal portion and/or a distal portion of the gastrointestinal system. More specifically, as discussed below, a proximal portion may include the duodenum and/or a proximal portion of the jejunum) and a distal portion may include the ileum proximate to the cecum, the cecum and/or the ileocecal valve.

As described in more detail below, an exemplary method implemented by the device 110 may stimulate a proximal site 103 more or less frequently than a distal site 105. Such a method may act to conserve power of an implantable device, noting that such a device may be rechargeable or have a replaceable power source. A rechargeable device may rely on an external power source that transmits energy to charge a capacitor or other storage.

The exemplary scenario 104 includes two or more implantable devices 112, 114. In this example, the device 112 senses, activates and/or blocks activity associated with a proximal portion of the gastrointestinal system 103. The other device 114 senses, activates and/or blocks activity associated with a distal portion of the gastrointestinal system 105. In general, the devices 112, 114 operate in a coordinated manner to delivery therapy to induce release of GLP-1 to improve cardiac condition. The devices 112, 114 may include circuits to allow for uni-directional or bi-directional communication. Pseudo-communication may occur via a circuit in one of the devices that detects electrical or other actions of the other device. For example, where the device 112 delivers energy to the stomach, the device 114 may sense the electrical activity via one or more electrodes or other circuits. The device 114 may rely on such information to determine appropriate action (e.g., sensing, activating, blocking, alerting, communicating, etc.).

The exemplary scenario 106 includes the implantable device 112 alone while the exemplary scenario 108 includes the implantable device 114, hence, if combined, the exemplary scenarios 106 and 108 are the same as the scenario 104.

Any of the scenarios 102, 104, 106, 108 may include an external device configured to acquire information from an implantable device and/or acquire information about the body 101. For example, an external device may acquire body weight information and then communicate this information to the implantable device 112 and/or 114. In turn, the implantable device 112 and/or 114 may use the communicated information to determine an action related to one or more proximal sites 103 and/or one or more distal sites 105.

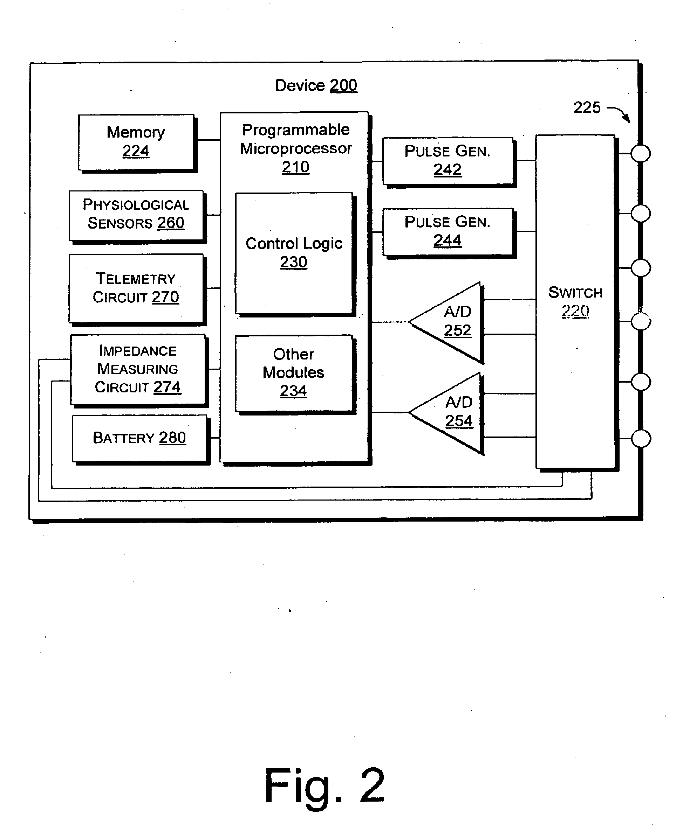

Fig. 2 shows a block diagram of an exemplary device capable of sensing, activating and/or blocking activity of any number of organs, muscles and/or nerves associated with metabolism and/or cardiac condition. A basic device may include a processor, memory, one or more inputs, one or more outputs and control logic stored as instructions in the memory and operable in conjunction with the processor. The device 200 includes various additional features.

The exemplary device 200 includes a programmable microprocessor 210 that can implement control logic 230 and other instructional modules 234. Information may be stored in memory 224 and accessed by the programmable microprocessor 210. For delivery activation energy, the device 200 includes one or more pulse generators 242, 244. The pulse generators 242, 244 may rely on a switch 220 for delivery of energy via one or more connectors 225. While a device may include one or more integral leads, in general, a device includes one or more connectors for connecting a lead or leads to the device. More particularly, the connectors 225 provide for electrically connecting one or more electrodes to the circuitry of the device 200. In the example of Fig. 2, the switch 220 may select an appropriate electrode configuration. An electrode configuration may include an electrode from one lead and an electrode from another lead, a case electrode and another electrode or electrodes from a single lead.

The device 200 further includes one or more analog to digital converters 252, 254 for converting analog signals to digital signals or values.
The processor 210 may use a signal provided by one of the A/D converters 252, 254 to control a therapy or other process. A control signal from the processor 210 may instruct the switch 220 to select a particular electrode configuration for sensing electrical or other activity. As discussed below, various techniques include sensing nerve activity or other activity.

The device may include one or more physiological sensors 260. Such sensors may be housed within a case of the device 200 (e.g., a motion sensor), include a surface mounted component, include a lead, include a remote sensor, etc. A sensor may provide a digital signal or an analog signal for use by the processor 210 or other circuitry of the device 200. A physiological sensor may provide a signal via one or more of the connectors 225. A physiological sensor may provide information on cardiac condition and/or cardiac performance. For example, a pressure sensor may provide information as to cardiac output.

For purposes of communication with external or other implantable devices, the device 200 includes a telemetry circuit 270. The telemetry circuit 270 may include one or more antennae for transmission and/or receipt of electromagnetic signals. Such a circuit may operate according to a specialized frequency or frequencies designated for medical devices. Various conventional implantable devices rely on an associated programmer, which is an typically an external computing device with a communication circuit suitable for communicating with an implantable device for purposes of data transfer, status checks, software download, etc. Where the circuit 270 communicates with an implantable device or a device in electrical connection with a patient's body, then the body may be a conductive medium for transfer of information. For example, the circuit 270 may be capable of communication with a specialized wristwatch where the body is relied upon as a conductor.

The device 200 further includes an impedance measuring circuit 274. Such a circuit may rely on instructions from the processor 210. For example, the processor 210 may instruct the circuit 274 to provide a measured impedance for a particular electrode configuration. In such an example, the processor 210 may also instruct the switch 220 to provide the circuit 274 with a particular electrode configuration. Impedance information may be used by the processor 210 for any of a variety of purposes (e.g., hardware condition, cardiac condition, edema, respiration, etc.). The processor 210 may store impedance or other information to memory 224 for later use or for transmission via the telemetry circuit 270.

The device 200 includes a power source, which is shown as a batter 280 in the example of Fig. 2. The battery 280 powers the processor 210 and optionally other circuitry, as appropriate. In general, the battery 280 provides power to the pulse generators 242, 244. Consequently, the battery 280 provides for operation of circuitry for processing control logic, etc., and provides for energy to activate tissue. A lead-based sensor may connect to the device 200 via one or more of the connectors 225 and be powered by the battery 280. The battery 280 may be rechargeable, replaceable, etc.

While the device 200 includes particular features, various exemplary devices, systems, methods, etc., may use or be implemented using a different device with more or less features.

### Exemplary Methods

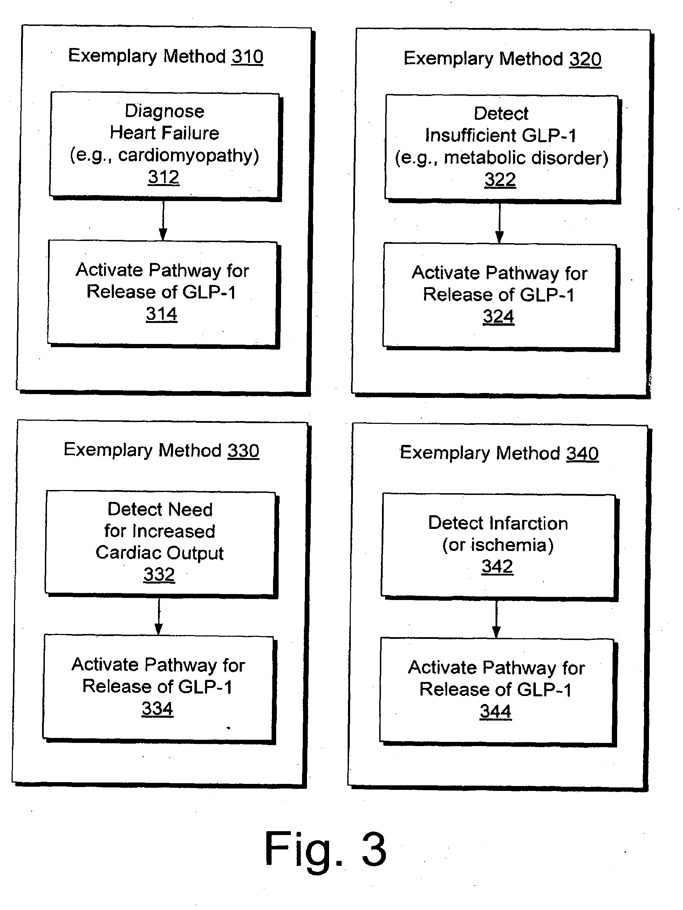

Fig. 3 shows various exemplary methods 310, 320, 330 and 340. The method 310 includes a diagnosis block 312 that diagnoses HF and an activation block 314 that activates a pathway for release of GLP-1 to treat HF. For example, the method 310 can include diagnosing HF and activating vagal nerves, K cells and/or L cells using an implantable device to induce, directly or indirectly, release of GLP-1 to treat heart failure. With respect to diagnosis of HF, the block 312 may rely on information acquired by an external device and/or an implantable device. Once a patient has been diagnosed with HF, the activation block 314 may operate to increase the patient's GLP-1 blood concentration beyond what it would be without intervention.

A study by Taegtmeyer indicates that GLP-1 can dramatically improve left ventricular function, systemic and coronary flow hemodynamics in canines with advanced cardiomyopathy ("Cardiac Metabolism as a Target for the Treatment of Heart Failure", Circulation 2004; 110: 894-896). Accordingly, the method 310 can treat human patients experiencing cardiomyopathy. Further, a study by Nikolaidis et al. ("Recombinant Glucagon-Like Peptide-1 Increases Myocardial Glucose Uptake and Improves Left Ventricular Performance in Conscious Dogs With Pacing-Induced Dilated Cardiomyopathy" Circulation 2004 110: 955-961) indicates that administration of recombinant GLP-1 (rGLP-1) to canines with pacing induced dilated cardiomyopathy can improve left ventricular performance. Hence, the exemplary method 310 may be implemented in conjunction with a cardiac pacing therapy to reduce risk of pacing-related cardiomyopathy.

In a specific example, the diagnosis block 312 acquires impedance information related to ventricular myopathy and then makes a diagnosis based at least in part on the acquired information. In turn, the activation block 314 uses the diagnosis to appropriately time activation of a pathway to increase GLP-1 concentration. In this example, the activation block 314 may operate according to a schedule. A schedule may be based directly on acquired information or on a diagnosis (e.g., based on the acquired information).

The exemplary method 320 includes a detection block 322 that detects insufficient GLP-1 and an activation block 324 for activating a pathway to release GLP-1. The detection block 322 may detect levels of GLP-1 directly or indirectly. For example, plasma gGLl concentration can be used as an indicator of GLP-1 concentration. Thus, the detection block 322 may rely on one or more gGLI concentration measurements. The method 320 can act to maintain a particular GLP-1 blood concentration or gastrointestinal concentration. In some instances, GLP-1 set point may be dependent on time of day or patient activity. In such instances, the activation block 324 may act by comparing a determined GLP-1 concentration to a set point concentration where the set point depends on one or more other variables. A concentration that is below a set point can be considered a low concentration. In general, a low concentration is a concentration less than a desired concentration.

While the detection block 322 refers to GLP-1, in another example, a detection block may detect blood glucose and use blood glucose (e.g., concentration) to determine insufficiency of GLP-1. Further, while the detection block 322 mentions insufficiency, an exemplary method may call for action where GLP-1 sufficiency exists (e.g., to prohibit delivery of energy to a pathway, to call for an adjustment to a cardiac pacing therapy, etc.).

The exemplary method 330 includes a detection block 332 that detects a need for increased cardiac output and an activation block 334 that activates a pathway for release of GLP-1. The method 330 can include detecting a need for increased cardiac output and calling for delivery of energy to the distal ileum to activate L cells using an implantable device where activation of L cells causes release of GLP-1 and where release of GLP-1 increases cardiac output. In general, such a method includes activating vagal nerves, K cells and/or L cells using an implantable device to induce, directly or indirectly, release of GLP-1 to increase cardiac output.

The aforementioned study by Nikolaidis et al. states that 48 hours of infusion of GLP-1 can increase cardiac output up to 27%. More specifically, the Nikolaidis study found that rGLP-1 significantly increased LV systolic pressure by about 9 mm Hg and LV dP/dt by about 960 mm Hg/s while reducing the LV end-diastolic pressure and heart rate. The Nikolaidis study also found that rGLP-1 significantly increased LV ejection fraction (from 28±1% to 38±5%), SV (14±3 mL), and CO (548±39 mL/min) while lowering systemic vascular resistance.

In a specific example, the detection block 332 acquires left atrial pressure information related to cardiac output (e.g., using a left atrial pressure sensor). In turn, the activation block 334 uses the detected information appropriately time activation of a pathway to increase GLP-1 concentration. In this example, the activation block 334 may operate according to a schedule. A schedule may be based directly on acquired information. For example, where left atrial pressure falls below a set point pressure (i.e., a low pressure for purpose of control), the activation block 334 may call for delivery of energy to a pathway for release of GLP-1 where the delivery occurs over a period of time according to a schedule. In another example, the delivery of energy may be adaptively adjusted based on left atrial pressure or other cardiac output information (e.g., on a beat-by-beat or another substantially instantaneous basis).

The exemplary method 340 includes a detection block 342 for detecting infarction or ischemia and an activation block 344 for activating a pathway for release of GLP-1. A study by Bose et al. ("Glucagon-Like Peptide 1 Can Directly Protect the Heart Against Ischemia/Reperfusion Injury", Diabetes, Vol. 54, January 2005) found that for induced ischemia in rats, GLP-1 infusion reduced infarct size by about 23%. In the method 340, the detection block 342 may detect infarct or ischemia by any of a variety of techniques and then trigger the activation block 344 to activate a pathway to increase GLP-1 concentration. The activation block 344 may operate according to a schedule, for example, based on severity of the cardiac event (e.g., magnitude, location, etc.).

In a particular example, the detection block 342 acquires temperature information using one or more temperature sensors positioned to measure blood and/or myocardial temperature. For example, a lead with temperature sensors may be positioned in the coronary sinus and/or its contributory vessels. In this example, an abnormal temperature or temperature differential may indicate that ischemia exists or that an infarct occurred. Accordingly, the activation block 344 may act to increase GLP-1 blood concentration. In another example, the detection block 342 acquires one or more intracardiac electrograms (IEGMs) and analyzes such information to decide whether an infarct or ischemic event occurred. The activation block 344 may then act accordingly to increase GLP-1 blood concentration.

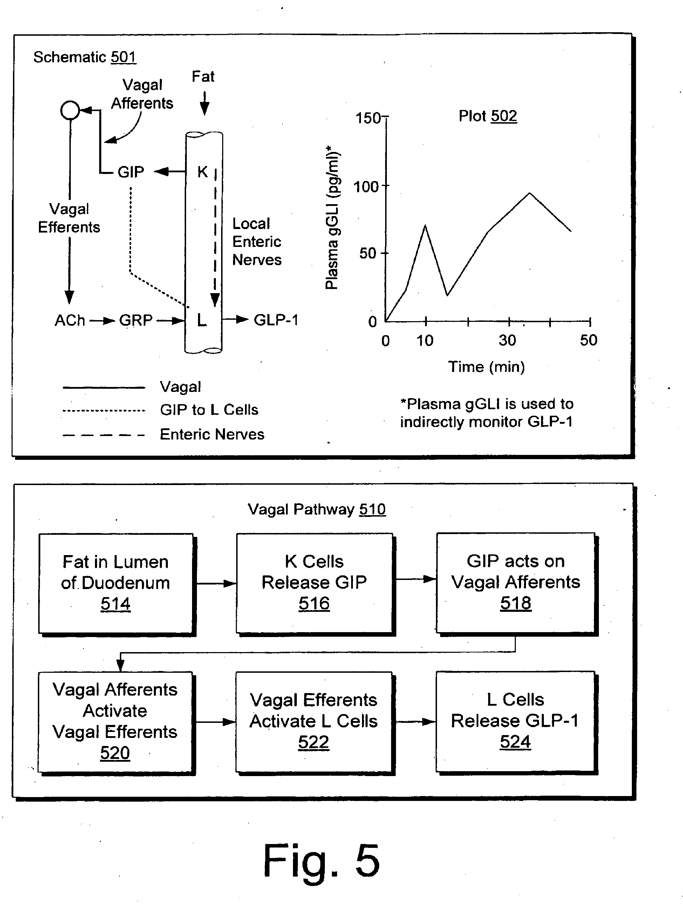

Accordingly, the method 340 can diminish long-term myocardial damage caused by an infarct or ischemia by detecting an infarct or ischemia and delivering energy to a site using an implantable device where activation at the site causes release of GLP-1 and where release of GLP-1 diminishes long-term myocardial damage caused by an infarct or ischemia. Such a method may deliver energy to one or more sites. For example, a method may deliver energy to one or more of a duodenal site, an ileum site, a vagal site, etc., as explained with respect to Fig. 5.

### Anatomy and Pathways

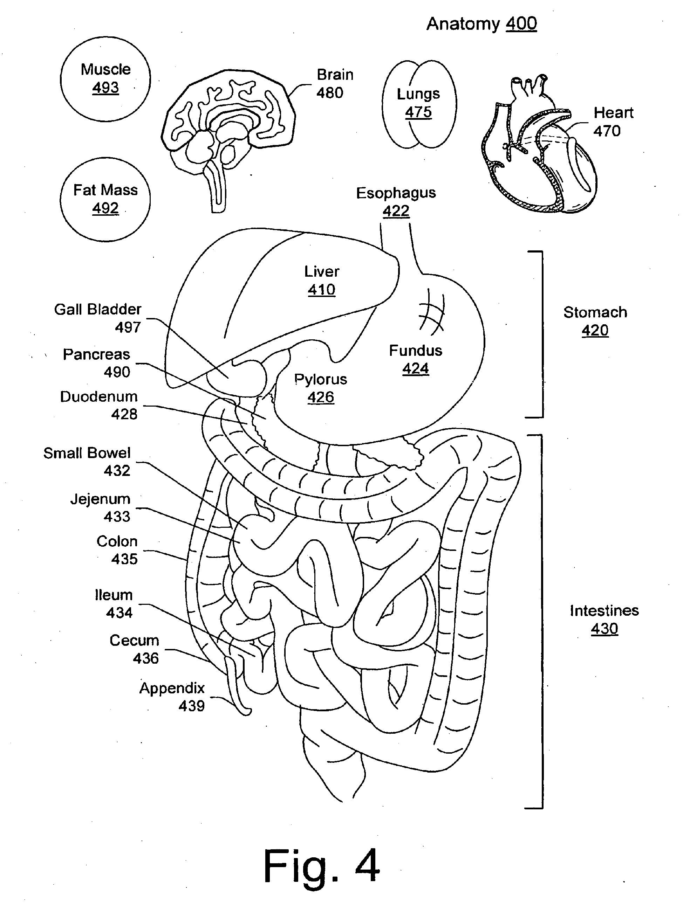

Fig. 4 is an anatomical diagram 400 of various organs and tissues involved in metabolism. The diagram 400 includes the liver 410, the stomach 420, the intestines 430, the heart 470, the lungs 475, the brain 480, the pancreas 490, fat mass 492, muscle 493 and the gall bladder 497. The stomach 420 includes labels that identify approximate locations of the esophagus 422, the fundus 424 and the pylorus 426. The intestines 430 include labels that identify approximate locations of the duodenum 428, the small bowel 432 (including the jejunum 433 and ileum 434) and the colon 435 (including the cecum 436). The appendix 439 is also identified. Neural pathways to the various organs and tissues, while not shown in Fig. 4, are discussed below with reference to Fig. 4.

Various studies refer to a "brain-gut axis" as a primary mechanism for metabolic control. A review by Konturek et al., "Brain-gut Axis and Its Role in the Control of Food Intake", J Physiol. Pharma. 2004, 55, 1, 137-154, recognizes that the gastrointestinal system or "tract" (GIT) interacts with the central nervous system (CNS) and the enteric nervous system (ENS) where the CNS and ENS form the brain-gut axis.

The ENS includes specific innervations and plexuses associated with the GIT. For example, the GIT includes a myenteric plexus known as the Auerbach plexus and a submucous plexus known as the Meissner plexus.
These plexuses are primarily associated with parasympathetic aspects of the autonomic nervous system. The Auerbach plexus is located between the longitudinal and circular layers of muscle in the tunica muscularis (generally from pylorus 426 to duodenum 428) and relates generally to tonic and rhythmic contractions. The Meissner plexus is buried in the submucosa and relates to epithelial cell function and GIT blood flow. Another plexus, the celiac plexus is associated primarily with sympathetic aspects of the autonomic nervous system, however, some vagal innervation exists as well as other innervation.

As already inferred, interactions exist between these ENS plexuses and the autonomic nervous system, in particular, a vagal pathway and a splanchnic path exist between the CNS and the ENS. The parasympathetic vagal pathway includes cholingeric nerves (about 80% to 90% unmyelinated C-fibers) while the sympathetic splanchnic pathway includes noradrenergic nerves. Regarding interactions, consider that the Valsalva maneuver, commonly associated with parasympathetic activation, as well as increasing intraabdominal pressure (e.g., squat position) can increase normal defecation.

With respect to more specific routes of autonomic innervation, more generally, vaso-vagal reflexes include sensory vagal afferents, second-order integrative neurons of the nucleus of the solitary tract (NTS) and efferent vagal neurons of the dorsal or posterior motor nucleus (DMN). Various studies indicate that the left vagus supplies efferent preganglionic fibers mainly to the anterior surface of the stomach (also known as the anterior vagus) and that the right vagus supplies efferent preganglionic fibers mainly to the posterior surface of the stomach (also known as the posterior vagus). For example, with reference to the diagram 400 of Fig. 4 the anterior vagus tracks from the esophagus 422 to the liver 410, the stomach 420 and the intestines 430. In addition both vagi send branches to the celiac plexus 462. The parasympathetic preganglionic vagal fibers penetrate the layers of the gastric wall, to form synapses in the ganglion cells of the Auerbach plexus and in the Meissner plexus. Postganglionic fibers emerge from the plexuses and supply musculature and mucosa.

The celiac plexus 462 is a junction for autonomic nerves supplying the upper abdominal organs (liver 410, gall bladder 497, spleen, stomach 420, pancreas 490, kidneys, small bowel 432, and about two-thirds of the large bowel or colon 435). The celiac plexus 462 proper consists of the celiac ganglia with a network of interconnecting fibers. The aorticorenal ganglia are often considered to be part of the celiac ganglia, and thus, part of the plexus. The celiac plexus 462 receives sympathetic nerves from the greater splanchnic nerve (around T5/6 to T9/10 vertebrae), the lesser splanchnic nerve (around T10/11 vertebrae) and the least splanchnic nerve (around T11/12 vertebrae). As already mentioned, the upper abdominal organs receive their parasympathetic supply from the left and right vagal trunks which pass through the celiac plexus 462.

The brain-gut axis also relies on afferent signals from the GIT and other organs. Afferent signals from the upper GIT are transmitted by the vagi, splanchnic mesenteric nerves, and pelvic afferents. Together, these afferent nerves provide the peripheral extrinsic neural part of the gut-brain axis. The upper GIT is largely innervated by vagal afferents; the pelvic afferents are limited to the lower or large bowel, while splanchnic afferents innervate much of the GIT. The intrinsic, enteric neural systems such as the myenteric and submucosal plexuses (Auerbach and Meissner) may mediate between GIT mucosal and muscular events and extrinsic neural signaling.

The vagus nerve consists of both sensory and motor axons. In the periphery, as already mentioned, the vagus enters the abdomen with two trunks (the right, dorsal or posterior and the left, ventral or anterior) that track generally along the esophagus. When the vagi cross the diaphragm, in most individuals they divide into five distinctive branches: (i-ii) paired gastric branches (e.g., associated with the stomach and the proximal duodenum), (iii-iv) paired celiac branches (e.g., associated with duodenum, jejunum, ileum, cecum and colon) and (v) a single hepatic branch that originates from the ventral trunk (e.g., associated with distal antrum, duodenum, liver and gall bladder).

With respect to the diagram 400 of Fig. 4 gastric braches of the of the anterior vagus 460 include direct branches to the fundus 424 (V-direct), pyloric branches to the pylorus 426 (branches emanating from vagal supply to liver 410 that include superior pyloric nerves and inferior pyloric nerves), a hepatic branch or branches (V-hepatic) and the anterior nerve of Latarjet (V-Latarjet, principal anterior nerve of lesser curvature of the fundus 424). Again various branches are linked to the aforementioned Auerbach plexus and Meissner plexus.

With respect to the heart 470, various autonomic pathways are discussed in an article by Kawashima ("The autonomic nervous system of the human heart with special reference to its origin, course, and peripheral distribution", Anat Embryol. (2005) 209: 425-438) and an article by Pauza et al. ("Morphology, distribution, and variability of the epicardiac neural ganglionated subplexuses in the human heart", The Anatomical Record (2000) 259(4): 353-382). The article by Kawashima presents various nerve pathways including parasympathetic cardiac branches arising from vagus nerve. The Kawashima article categorizes vagal cardiac branches with direct connections or connections via the cardiac plexus, excluding branches of the lung or surrounding vessels and organs, as follows: superior cardiac branch, which arose from the vagus nerve at about the level of the upper (proximal) portion of the recurrent laryngeal nerve branch inferior cardiac branch, which arose from the recurrent laryngeal nerve branch; and thoracic cardiac branch, which arose from the vagus nerve at about the level of the lower (distal) portion of the recurrent laryngeal nerve branch.

According to Kawashima, the cardiac plexus includes the right cardiac plexus, which usually surrounds the brachiocephalic trunk (also known as the innominate artery), and the left cardiac plexus, which surrounds the aortic arch. On the right side, several autonomic nerves were observed passing through the dorsal, rather than the ventral, aspect of the aortic arch while, on the left side, no differences between the ventral and dorsal courses to the aortic arch were observed.

Various nerves identified in the Kawashima article extend to one or more epicardial autonomic plexuses, also referred to herein as subplexuses. The aforementioned article by Pauza et al., reports that the epicardial plexus includes seven subplexuses: (I) left coronary, (II) right coronary, (III) ventral right atrial, (IV) ventral left atrial, (V) left dorsal, (VI) middle dorsal, and (VII) dorsal right atrial. The Pauza article states that, in general, the human right atrium is innervated by two subplexuses (III, VII), the left atrium by three subplexuses (IV, V, VI), the right ventricle by one subplexus (II), and the left ventricle by three subplexuses (I, V, VI).

Various autonomic pathways associated with the cardiopulmonary system innervate the heart 470 (e.g., cardiac subplexuses), the brain 480, the lungs 475, and baroreceptors (e.g., such as those associated with the aortic arch). While some parasympathetic pathways may operate without direct communication to the brain 480, the brain often activates efferent pathways and receives information via afferent pathways.

As parasympathetic pathways connect with various organs, more than one type of nerve activity exists for communication along such parasympathetic pathways. Further, the specific type of activity may, by itself, identify an associated mechanism or group of mechanisms. For example, sensing of high velocity nerve activity may indicate that respiratory and/or baroreceptor mechanisms are involved. A classification method may optionally rely on sensing nerve activity and determining the type of nerve activity (e.g., velocity, frequency or other characteristics) to then classify the activity as associated with a mechanism or organ. Classification may occur based at least in part on one or more other types of information. For example, electrocardiogram, impedance and pressure information may be used for purposes of classifying nerve activity.

### Incretin Effect

The incretin effect, defined by a significantly greater insulin stimulatory effect evoked after an oral glucose load than that evoked from an intravenous glucose infusion when plasma glucose concentrations are matched, was first described in the 1960s. Although other hormones may take part in the incretin effect, the majority of the effect is due to glucose-dependent insulinotropic peptide (GIP) and GLP-1. Patients with type 2 diabetes have a significant reduction of the incretin effect, implying that these patients either have decreased concentration of the incretin hormones, or a resistance to their effects. GLP-1 concentrations are reduced in patients with type 2 diabetes in response to a meal, while GIP concentrations are either normal or increased, suggesting a resistance to the actions of GIP thus making GLP-1 a more logical target for therapeutic intervention.

The incretin effect is estimated to be responsible for about 50% to about 70% of the insulin response to glucose. As already mentioned, in patients with type 2 diabetes, the incretin effect is diminished or absent. This reduction in the incretin effect is due in part to a partial reduction in postprandial GLP-1 secretion and a substantial reduction in the insulinotropic activity of GIP. The incretin effect causes a fairly rapid increase in plasma insulin, usually within about the first 30 minutes after ingestion of glucose. Patients with type 2 diabetes may eventually reach a similar plasma insulin value after, for example, at about 120 minutes.

Referring to the activation blocks 314, 324, 334 and 344 of Fig. 3, such blocks can aim to mimic at least part of the incretin effect and thereby cause release of GLP-1. As described herein, delivery of electrical energy to a portion or portions of the gastrointestinal system may act to increase secretion of GLP-1. For example, as described further below, GLP-1 is secreted by so-called "L" cells in the distal ileum, under some degree of control by enteric cholinergic neurons. Delivery of electrical energy to L cells can cause release of GLP-1. In turn, GLP-1 can act in various manners such as on the pancreas to cause release of insulin from beta cells.

The hormone GIP can enhance secretion of GLP-1. GIP is secreted by so-called "K" cells in the proximal duodenum, under some degree of control by enteric cholinergic neurons. Delivery of electrical energy to K cells can cause release of GIP. In turn, GIP acts to increase release of GLP-1.

Referring again to Fig. 1, in the various arrangements, a proximal site 103 may correspond to a site to promote release of GIP while a distal site 105 may correspond to a site to promote release of GLP-1. Various exemplary methods may include (i) gastric neurostimulation to stimulate release of GIP and GLP-1 as a cardiac therapy, (ii) gastric neurostimulation to stimulate release of GIP from the duodenum as a cardiac therapy and/or (iii) gastric neurostimulation to stimulate release of GLP-1 from the ileum as a cardiac therapy.

### Direct and Indirect Pathways for Inducing Release of GLP-1

Evidence based on anterograde tracing after injections into the individual subdiaphragmatic vagal branches show discrete, yet somewhat overlapping, NTS termination fields for each vagal branch. Thus, the NTS contains viscerotopic and at least some information specific to a particular vagus branch. As already discussed, the hepatic branch of the anterior vagus includes branches that supply other regions. Other branches also exist and lead to the antrum, the duodenum, and the cecum, which is consistent with the general notion that individual gut vagal branches innervate multiple GIT segments.

With respect to efferent nerves, various efferent nerves project from the DMN, a structure just anterior of the NTS. The DMN contains numerous dendrites penetrating the NTS and the Area Postrema (AP). About 95% of preganglionic neurons in the DMN contribute to projections to the stomach suggesting that the efferent part of the vagus nerve is highly involved in the motor innervation of the stomach. Anterograde tracers injected into the DMN have been shown to label up to 100% of the myenteric ganglia in the stomach, 96% in the duodenum, 40% in the jejunum, 66% in cecum, 16% in the descending colon, and 0% in the rectum.

The posterior vagus appears to have more efferent character while the anterior vagus appears to have more afferent character with respect to the GIT. Hence, as in the afferent projections, each vagal branch contains the axons of a topographically distinct column of cells within the DMN. Therefore, the afferent-efferent viscerotopic and branch specific organization of the vagus likely reflects distinct neurophysiological reflexes and functions involved in digestion. Such an organization may be used to further the goals of various exemplary techniques discussed herein.

Vagal afferents appear to be sensitive to a variety of stimuli. Different populations of vagal afferents with terminations in the GIT indicate different sensory modalities, for example, reaction to mechanical as well as chemical stimulation. More specifically, vagal endings in the longitudinal and circular muscle layers are also defined as intramuscular arrays (IMAs) and have been suggested to be in-series tension receptors (e.g., mechanical sensors).

Vagal afferents in the myenteric plexus (Auerbach) throughout the GIT are called intraganglionic laminar endings (IGLEs) and exhibit characteristics of a tension receptor. Thus, evidence indicates that vagal afferent fibers play a role in volume detection of the stomach and gut, which may contribute to the process of satiation. This role is supported by neurophysiological studies that link the vagus to the process of satiation and meal termination.

With respect to the paired celiac branches of the vagus, motor fibers of the accessory celiac and celiac vagal branches are derived from the lateral columns of the DMN. These branches also contain sensory fibers that terminate within the NTS. Studies on rats report that tracer injections of the duodenum labeled a small number of the cells within the medial aspects of the DMN, that jejunal, ileal, and ascending colon injections labeled cells sparsely within the lateral aspects of the DMN bilaterally, that cecal injections caused prominent retrograde labeling of cell bodies bilaterally in the lateral columns of the DMN above, at, and below the level of the area postrema and that no afferent terminal labeling was evident after injection of these areas of the bowel.

Studies in humans (see, e.g., Wettergren et al., The inhibitory effect of glucagon-like peptide-1 (GLP-1) 7-36 amide on gastric acid secretion in humans depends on an intact vagal innervation", Gut 1997;40;597-601) report that GLP-1 does not act directly on the gastric mucosa, but interacts with vagal pathways (e.g., receptors associated with vagal afferents, central nervous sites, or transmission in vagal efferents). The study of Wettergren reported that GLP-1 probably does not interfere with vagal efferent transmission at the level of the parasympathetic ganglia and that GLP-1 released from the small intestine could reach the brain via leaks in the blood-brain barrier.

Studies indicate that both vagal and spinal afferent pathways play a role in mediating nutrient-specific information from the GIT. Vagal afferent pathways transmit information about protein/amino acids (AAs), lipids, and carbohydrates (hexoses); for the latter, the vagal afferents seem to be in the accessory celiac branch of the vagus. In addition, spinal afferents transmit information for hexoses, but not necessarily for lipids, via the celiac plexus and the dorsal root ganglion (or spinal ganglion). Activation of both spinal and vagal afferent fibers results in alteration of autonomic efferent outflow to alter secretory and motor function of the GIT.

Fig. 5 shows a schematic 501, a plot 502 and a flowchart 510 for the mechanism proposed by Rocca and Brubaker, which suggests that physiological doses of GIP act through the nervous system (either vagal or myenteric) to indirectly stimulate GLP-1 secretion, rather than acting directly at the level of the L cell. The schematic 501 and the flowchart 510 show fat in the lumen of the duodenum 514 as stimulating the release of GIP from the K cells 516. GIP released by K cells acts on vagal afferents 518, which, in turn, activate vagal efferents 520. The vagal efferents then activate the L cells 522 and the L cells release GLP-1. Rocca and Brubaker report that the proximal signal is mediated by vagal efferent pathways present in the celiac branch of the subdiaphragmatic vagus nerve that are thought to synapse with GRPergic neurons present in the myenteric plexus. At supraphysiological concentrations, GIP may also act directly through receptors on the L cell (dotted line in the schematic 501). Mechanisms that stimulate early secretion may also be mediated through the enteric nervous system in the walls of the gastrointestinal tract (dashed line in the schematic 501).

Rocca and Brubaker suggest that secretion of GLP-1 from the distal L cell is intimately connected with the presence of nutrients in the proximal duodenum through an interaction of neural and endocrine pathways. Rocca and Brubaker performed electrical stimulation of the celiac branch of the vagus nerve in rats to determine whether the efferent vagus nerve mediates the signal from the proximal duodenum to the distal L cells. The plot 502 shows responses of plasma gGLI (as difference from basal) to electrical stimulation of the distal end of the celiac branch of the vagus nerve. The electrical stimulation used a potential of 10 V at a frequency of 20 Hz for a period of 15 minutes. Rocca and Brubaker also suggest that the enteric nervous system is not responsible for relaying information about nutrient intake to the ileal L cells. They further suggest that local afferents could possibly branch from vagal afferents that innervate the duodenal region and travel to the ileum within the walls of the gastrointestinal tract (see dashed line for enteric nerves, which may include local vagal afferents) to exert effects on gGLI secretion in a reflex manner. Thus, multiple mechanisms may exist for transmission of information (e.g., "global" and local).

### Chemical Messengers

Before discussing various exemplary methods for inducing release of GLP-1, various chemical messengers (GIP, GLP-1, insulin and glucagons) are discussed in more detail. GIP, also known as gastric inhibitory polypeptide, or glucose-dependent insulinotropic polypeptide, is a 42 amino acid peptide hormone synthesized in and secreted from K cells in the intestinal epithelium. The majority of intestinal K cells are located in the proximal duodenum. GIP secretion is primarily regulated by nutrients, especially fats. GIP exhibits potent incretin activity in rodents and human subjects. Unlike GLP-1, which exerts multiple non-incretin activities in the regulation of blood glucose, the primary action of GIP is the stimulation of glucose-dependent insulin secretion. GIP may also play a role in adipocyte biology. The site of GIP expression, the gut endocrine K cell, possess features for sensing nutrient intake and secreting GIP following nutrient intake. GIP has been shown to stimulate beta cell proliferation synergistically with glucose in the islet INS-1 cell line. Thus, potential exists for use of GIP in combination with GLP-1 for initiating islet regeneration in the setting of islet injury or decrease beta cell mass. GIP receptors are expressed on adipocytes.

GLP-1 is an incretin that is essential for normal glucose tolerance and is a product of proglucagon that is secreted by specialized intestinal endocrine cells (L cells) after meals. The majority of circulating biologically active GLP-1 is found in the GLP-1 (7-36) amide form, with lesser amounts of the bioactive GLP-1 (7-37) form also detectable. It is the first insulinotropic GI hormone to be identified that is effective in diabetic as well as nondiabetic persons. The effect of GLP-1 to lower blood glucose is likely due to effects on several processes, including gastric motility, food intake, and possibly glucose production and/or glucose uptake, as well as islet hormone secretion (e.g., insulin). Studies suggest that GLP-1 is involved in the regulation of beta cell mass, for example, GLP-1 signaling appears to regulate islet proliferation and islet neogenesis (e.g., can cause beta cell proliferation). At least some of these processes appear to be mediated through visceral neural pathways (e.g., at least gastric emptying and satiety).

Various studies show that GLP-1 enters the blood and acts on the nervous system and the pancreas. GLP-1 stimulates insulin secretion from beta cells in the pancreas in a glucose-dependent manner when the glucose concentration is elevated, an effect that declines as glucose concentration returns toward normal. GLP-1 also acts via the nervous system to affect the pancreas. Insulin, as discussed below, acts to reduce glucose output by the liver and acts to increase uptake by muscle, including the myocardium. GLP-1 increases glycogenesis by the liver and muscle, a process of glycogen synthesis, in which glucose molecules are added to chains of glycogen. GLP-1 also acts to increase lipogenesis, a collective, complex process of producing lipids (fatty acids) from smaller precursor molecules such as glucose.

As GLP-1 can cause a decrease in glucose, it may indirectly increase release of somatostatin, which acts to suppress release of gastrointestinal hormones (e.g., gastrin, CCK, secretin, motilin, GIP, etc.), lowers the rate of gastric emptying and reduces smooth muscle contractions and blood flow within the intestine, suppress the release of pancreatic hormones (inhibits release of insulin and glucagon) and suppress the exocrine secretory action of pancreas.

GLP-1 has four main actions referred to as (i) pancreas insulin-related, (ii) glucagon secretion, (iii) satiety and (iv) gastric-related. Pancreas insulin-related action includes various actions discussed with respect to the schematic 501 where GLP-1 is released by L cells and acts on beta cells in proportion to the amount of glucose sensed by L cells.

The beta cells of the Islets of Langerhans of the pancreas produce insulin. Circulating insulin levels increase upon food intake and are proportional to body fat. Insulin promotes transport of glucose from the circulatory system into tissues, stimulates uptake of glucose and deposition of glycogen in the liver, decreases release of glucose by the liver, decreases food intake and increases energy expenditure via action on the hypothalamus and/or hindbrain.

Glucagon is a hormone that causes the release of glucose from the liver in order to maintain normal glucose homeostasis during fasting and is an important protective mechanism against hypoglycemia. Conversely, glucagon secretion is suppressed during periods of hyperglycemia. GLP-1 suppresses glucagon secretion from alpha cells in the pancreas in a glucose-dependent manner where more specifically it suppresses glucagon secretion in the fed state (noting during hypoglycemia, glucagon suppression does not occur). However, in patients with type 2 diabetes, glucagon concentrations are often inappropriately elevated, resulting in increased hepatic glucose output and postprandial glucose excursions.

With respect to satiety, the central nervous system is a critical site for regulating food intake, satiety, and body weight. GLP-1 works on the brain to trigger a feeling of satiety and reduce food intake.

With respect to gastric emptying, the rate of gastric emptying is a key determinant of the rate of nutrient absorption, an important factor in postprandial glucose excursions. GLP-1 slows the rate of gastric emptying, which in turn slows the delivery and absorption of nutrients in the small intestine and thereby limits the rate at which carbohydrate is absorbed into the circulation. The rate of gastric emptying is often increased in patients with type 2 diabetes, causing a rapid rise in postprandial blood glucose.

Type 2 diabetes is associated with an imbalance of beta cell response and beta cell workload. The response diminishes while workload increases. GLP-1 causes proliferation of beta cells and hence may prove more beneficial as a therapeutic approach when compared to therapies that use or propose electrical pancreas stimulation alone.

### Exemplary Methods

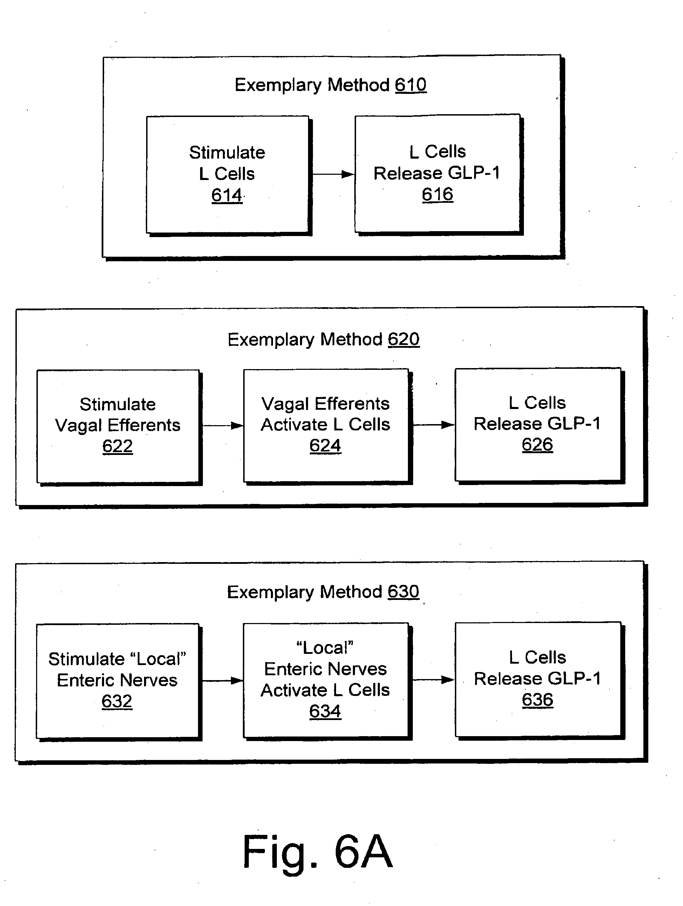

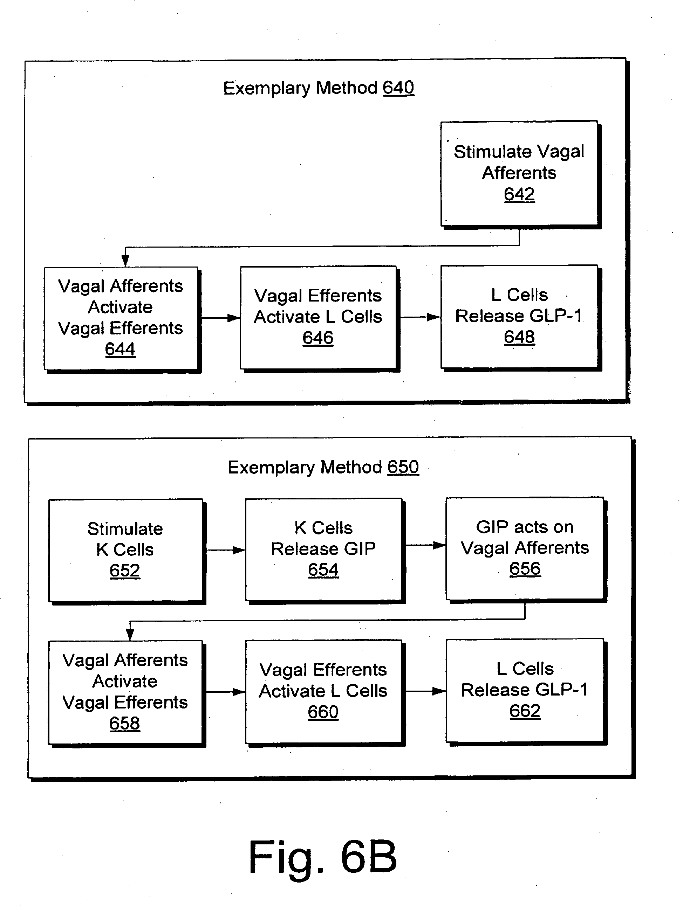

Figs. 6A and 6B show various exemplary methods 610, 620, 630, 640 and 650 that aim to induce release of GLP-1 to treat cardiac condition or to improve cardiac performance. Such methods may be part of the methods 310, 320, 330 or 340 of Fig. 3. For example, the method 610 may be performed by the activation block 314 of the method 310.

The exemplary method 610 includes a stimulation block 614 and a release block 616. Accordingly, the stimulation block 614 may directly stimulate L cells to thereby cause release of GLP-1, per the release block 616. With respect to direct L cell stimulation, general stimulation of adrenergic receptors stimulates secretory activity of ileal endocrine L cells where secretion results from the activation of the beta2-isoreceptor type. Additionally, GLP-1 secretions are positively modulated by alpha1-receptor stimulation and inhibited by alpha2-receptor activation upon beta-receptor occupation. While various techniques include delivery of electrical energy, an exemplary method may deliver a chemical to stimulate L cells.

The exemplary method 620 includes a stimulation block 622 for stimulation of vagal efferents. In turn, vagal efferents activate L cells 624 and L cells release GLP-1. The stimulation block 622 may stimulate vagal efferents at any of a variety of locations. Such stimulation may be via delivery of electrical energy directly to vagal efferents. As shown in the schematic 501 of Fig. 5, the vagal efferents may release ACh, which can act to release gastrin-releasing peptide (GRP). GRP is localized extensively in the myenteric plexus and potently stimulates the secretion of GLP-1. Rocca and Brubaker state that GRP is a likely candidate neuropeptide that may be released upon electrical stimulation of the celiac branch of the vagus nerve. Further, they note that delayed secretion of gGLI (see plot 502 of Fig. 5) in response to electrical stimulation is not dependent upon release of GIP from the duodenum, as vagal stimulation has previously been shown to have no effect on the GIP-containing K cell.

The exemplary method 630 includes a stimulation block 632 for stimulation of local enteric nerves. In turn, the enteric nerves activate L cells 634, which then release GLP-1 636. Rocca and Brubaker state that mechanisms that stimulate early secretion of GLP-1 may also be mediated through the enteric nervous system in the walls of the gastrointestinal tract. Thus, the method 630 may be used to induce the release of GLP-1 or to mediate the release of GLP-1.

The exemplary method 640 includes a stimulation block 642 for stimulation of vagal afferents. The stimulation block 642 may stimulate vagal afferents at any of a variety of locations. Such stimulation may be via delivery of electrical energy directly to vagal afferents. As shown in the schematic 501 of Fig. 5, the vagal afferents may act on the brain and subsequently on vagal efferents 644. In turn, vagal efferents activate L cells 646 and L cells release GLP-1 648. Vagal afferent pathways may exist locally. Rocca and Brubaker recognize that local afferents are extensions of vagal afferents that divide from the main afferent nerve and supply a target organ directly without synapsing with higher centers (e.g., the brain). Such afferents may branch from vagal afferents that innervate the duodenal region and travel to the ileum within the walls of the gastrointestinal tract to exert effects on gGLI secretion in a reflex manner; noting that gGLI levels are highly correlated with those of GLP-1.

The exemplary method 650 includes a stimulation block 652 to directly stimulate K cells. In turn, the K cells release GIP 654, which acts on vagal afferents 656. As already described, the vagal afferents cause vagal efferent activity 658 that acts on L cells 660, which release GLP-1 662. Various GIP secretagogues exist. Glucose, protein hydrolysates, specific amino acids, and fat stimulate GIP release by K cells. Further, GRP, a hormone produced and secreted by enteric neurons, also stimulates GIP release. Conversely, somatostatin (SST) inhibits secretion from gut K cells. While various techniques include delivery of electrical energy, an exemplary method may deliver a chemical to stimulate K cells.

While the method 650 pertains to a vagal afferent/efferent pathway for inducing release of GLP-1, GIP released by K cell stimulation may act in a more direct manner as indicated by the dotted line in the schematic 501 of Fig. 5. As explained with respect to Fig. 5, GIP stimulates GLP-1 secretion from gut L cells GIP, for example, by acting directly on receptors on L cells. Thus, an exemplary method may stimulate K cells to induce release of GIP to thereby induce release of GLP-1 through one or more pathways (e.g., afferent/efferent pathway and/or receptor on L cell pathway). Rocca and Brubaker noted that GIP at supraphysiological concentrations can induce GLP-1 release by the receptor on L cell pathway. More specifically, when infused at supraphysiological doses, GIP stimulated gGLI secretion significantly in both control and vagotomized animals; however, a vagal dependence of GIP became evident when GIP was infused at physiological doses. This indicates that the hepatic branch of the subdiaphragmatic vagus nerve mediates the stimulatory effect of GIP on the L cell. An exemplary method can delivery GIP or stimulate K cells to release GIP to produce a local supraphysiological concentration of GIP. Such a concentration of GIP can then cause L cells to secrete GLP-1 by at least the receptor pathway.

### Exemplary Sites

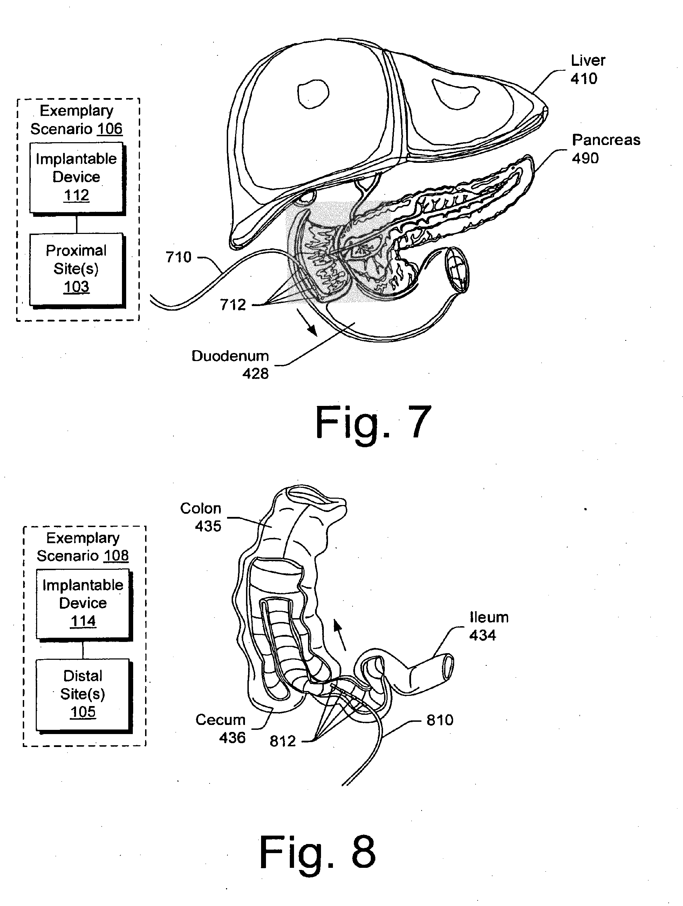

Figs. 7 and 8 show exemplary sites for delivery of electrical energy to a proximal site and a distal site, respectively. The small intestine is divided into three segments: duodenum 428, jejunum 433, and ileum 434. The proximal site of Fig. 7 corresponds to the duodenum 428, which, as already mentioned, includes K cells that secrete GIP and the distal site of Fig. 8 corresponds to the distal ileum 434, which, as already mentioned, includes L cells that secrete GLP-1.

The structure of the intestinal wall is not consistent throughout the entire small intestine. The duodenum 428 contains a thick wall, with deeply folded mucous membrane and duodenal digestive glands. The foldings are called the "folds of Kerckring". They are well developed both in the duodenum 428 and in the jejunum 433. The jejunum 433 has a thicker wall and is more vasculated than the duodenum 428. Furthermore, it has larger and more villi than the ileum 434. The ileum 434 has more lymphatic follicles than elsewhere in the intestine.

The small intestine has various layers including an outer layer referred to as the serosa and inner layers (e.g., muscularis, submucosa and mucosa). The myenteric plexus (plexus of Auerbach) is generally in the muscularis between a longitudinal layer of muscle and a circular layer of muscle.

Fig. 7 shows a lead 710 that includes a series of electrodes 712 implanted in the duodenum 428 subserosally. In the instance that the lead enters the lumen, the tip of the lead 710 may be positioned downstream, i.e., in the general direction of flow. While lead-based electrodes are shown, electrodes may be patch electrodes, paddle electrodes or other type(s) of electrodes, for example, as used for nerve stimulation. The electrodes 712 are electrically connected to an implantable device such as the device 200 of Fig. 2.

Fig. 8 shows a lead 810 that includes a series of electrodes 812 implanted in the distal ileum 434 subserosally. In the instance that the lead enters the lumen, the tip of the lead 810 may be positioned downstream, i.e., in the general direction of flow. While lead-based electrodes are shown, electrodes may be patch electrodes, paddle electrodes or other type(s) of electrodes, for example, as used for nerve stimulation. The electrodes 812 are electrically connected to an implantable device such as the device 200 of Fig. 2. Fig. 8 also shows the ileocecal valve (including intussusceptum and intussuscipens), which is situated at the junction of the ileum 434 and the colon 435. The appendix 439 is not shown, noting that surgical procedures to this region of the intestines is routinely performed using laproscopic and other techniques.

While the scenarios 106 and 108 of shown in Figs. 7 and 8 refer to delivery of electrical energy to various sites, an exemplary scenario may deliver a chemical where the chemical induces release, directly or indirectly, of GIP or GLP-1.

### Exemplary Activation Parameters

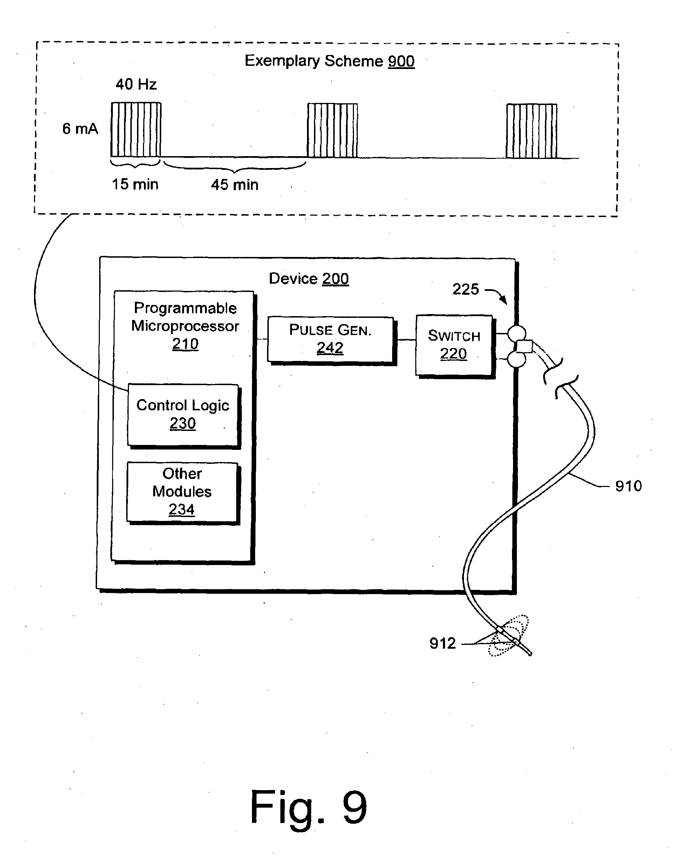

Fig. 9 shows an exemplary energy delivery scheme 900. According to the scheme 900, energy is delivered at approximately 6 mA at a frequency of approximately 40 Hz for a duration of approximately 15 minutes every hour (i.e., 15 minutes on and 45 minutes off). During the "on" time, the duty cycle is approximately 1%, for a pulse width of about 250 µs and a frequency of about 40 Hz. The "on" time may be selected in any of a variety of manners. For example, the "on" time may be selected with reference to the incretin effect; the incretin effect usually occurs within 30 minutes of glucose intake (see, e.g., plot 502 of Fig. 5) and gGLI reaches a peak within about an hour after the glucose intake.

The exemplary scheme 900 may be modified as appropriate. For example, where energy delivery occurs at 40 Hz for 15 minutes, the energy delivery may be further subdivided such as small bursts of 2 seconds on and then 2 seconds off, as opposed to continuous delivery at 40 Hz for the full 15 minutes. Thus, energy delivery may be characterized in terms of "on/off" times (e.g., "2 s/2 s"), which may cycle continually during an active therapy window (e.g., the 15 minute window) while energy delivery does not occur during an inactive window (e.g., the 45 minute window). Use of "on/off" times may occur for physiologic reasons. Where sensing occurs, such times may be optimized optionally together with active therapy window and/or inactive therapy window durations.

The scheme 900 may be implemented as control logic 230 of an implantable device 200 that may include a lead 910 with a series of electrodes 912 positionable at any of a variety of sites (e.g., digestive track sites, nerve sites, muscle sites, etc.). A lead optionally includes a branch or branches that may allow for positioning at least one electrode at a proximal digestive track site and at least one electrode at another site (e.g., a distal digestive track site, a nerve site, a muscle site, etc.). A lead optionally includes a branch or branches that may allow for positioning at least one electrode at a distal digestive track site and at least one electrode at another site (e.g., a proximal digestive track site, a nerve site, a muscle site, etc.).

Exemplary stimulation parameters are shown in Table 1 and may be, for example, of active biphasic or passive biphasic pulse morphology.

**Table 1. Exemplary Parameters**

| | | |
|---|---|---|
| Pulse Amplitude: | 0 mA - 10 mA | Typical : 4 mA - 6 mA |
| Pulse Width: | 150 µs - 500 µs | Typical : 250 µs |
| Pulse Frequency: | 10 Hz - 50 Hz | Typical : 40 Hz |
| Burst Duration: | 1 minute - 15 minutes | Typical : 15 minutes |
| Burst Period: | 1 minute - 60 minutes | Typical : 45 minutes |

With respect to charge density, limits may be imposed, for example, to not exceed about 100 µc/cm² charge density delivery to the tissue.

Table 2, below, provides examples of delivery schemes (S1 through S7) with reference to proximal site activation and distal site activation for various times (Time 1 through Time 4). The scheme S1 delivers energy in a manner that aims to primarily activate a proximal site ("P") while the scheme S2 delivers energy in a manner that aims to primarily activate a distal site ("D"). In contrast, schemes S3-S7 deliver energy in a manner that aims to activate both a proximal site and a distal site ("P+D"). In these examples, a proximal site may correspond to a site of the duodenum 428 as shown in Fig. 7 and a distal site may correspond to a site of the distal ileum 434 as shown in Fig. 8. In these examples, a proximal site may correspond to a site that includes K cells and a distal site may correspond to a site that includes L cells. In these examples, a proximal site may correspond to a mechanism that affects secretion of GIP and a distal site may correspond to a mechanism that affects secretion of GLP-1. As explained in Fig. 5, secretion of GIP may cause secretion of GLP-1.

**Table 2. Delivery Schemes**

| | Time 1 | Time 2 | Time 3 | Time 4 |
|---|---|---|---|---|
| S1 | P | P | P | P |
| S2 | D | D | D | D |
| S3 | P | D | P | D |
| S4 | P+D | P | P+D | P |
| S5 | P+D | D | P+D | D |
| S6 | P+D | P+D | P+D | P+D |
| S7 | P+D | P | D | P+D |

As noted in schemes S4-S7, energy may be delivered in a manner that aims to activate both a proximal site and a distal site at a particular time. Overall, if a method aims to affect, at some point in time, the muscles only, then a delivery scheme may be selected that minimizes its effect on C-fibers. Such a method may aim to reduce activation of C-fibers by reducing charge density delivered per pulse. In particular, the pulse width of the energy may be limited to a short duration (e.g., about 1 ms). Further, a delivery scheme that reduces charge density can reduce tissue damage. Selective activation can enhance control or effectiveness of various schemes.

As already mentioned, a site may be selected to activate/inhibit tissue activity (e.g., L cells, K cells, muscle, nerve, etc.). For example, Fig. 5 shows a mechanism that pertains to K cells, L cells, nerve cells and other tissue/organ cells. With respect to nerves, a nerve in the human body is typically composed of thousands of fibers, of different sizes, which may be designations by group (e.g., A, B and C). The vagus nerve, for example, may have approximately 100,000 fibers of various sizes (e.g., approximate diameters or cross-section) where each fiber can carry a signal. Normally, each axon (fiber) conducts in only one direction. Conduction velocity varies depending on size and other physiology such as myelination. The aforementioned A and B group fibers are myelinated, whereas C group fibers are unmyelinated.

Myelinated fibers are typically larger, conduct faster and have very low stimulation thresholds, compared to the unmyelinated type. Further, less energy is typically required to stimulate myelinated fibers, which exhibit a particular strength-duration curve that may relate to response for a given pulse width versus amplitude. In general, A and B group fibers can be stimulated with relatively narrow pulse widths (e.g., typically less than 1 ms). With respect to conduction, A group fibers conduct slightly faster than B group fibers and typically exhibit a slightly lower activation threshold. C group fibers are very small, conduct electrical signals very slowly, and have higher stimulation thresholds typically requiring a wider pulse width and higher amplitude for activation when compared to A and B group fibers. Hence, selective stimulation of only A and B group fibers may be achieved given appropriate equipment.

An exemplary method optionally includes control logic to classify sensed nerve activity where such classification may rely on groups such as A, B and C, myelinated/unmyelinated, etc. Activation of a nerve may rely on such classification. Activation may aim to transmit a signal or to block or inhibit a signal.

As described herein, inhibition or blocking of signals of a vagus nerve may be used for treating or controlling a metabolic disorder and/or treating a cardiac condition. Activation of a vagal nerve is accompanied by generation of a signal or signals, assuming the nerve or nerve fibers is/are not in a refractory phase. Some GIT signals are carried by C-fibers, which become refractory if stimulated at high frequency (e.g., about 40 Hz or higher) for more than a period of 30 to 60 seconds. Thus, an exemplary method may aim to inhibit or block C-fiber transmission by through use of a scheme that delivers energy using a high frequency with a certain "on" time followed by an "off' time (e.g., consider about 300 seconds on followed by about 20 seconds). This scheme could be repeated for the interval of time that control (blocking of the C-fiber information) is desired to be exercised.

An alternative scheme may consider that C-fibers become refractory if stimulated for a sufficiently long period. Thus, an alternative scheme may continuously stimulate the C-fibers to render them refractory and thereby block nerve signal transmission. Again, various GIT signals are carried by C-fibers, hence, such blocking schemes may be appropriate on a continuous, a periodic or an as desired/needed basis (e.g., in response to an event, a signal, a schedule, etc.). C-fibers conduct more slowly than A and B-fibers; an exemplary scheme may account for such conduction differences, particularly when blocking certain C-fiber signals.

As described herein, where an exemplary method aims to activate tissue other than C-fibers, a delivery scheme can optionally use energy delivery parameters that aim to minimize effect on C-fibers. Such a scheme may, for example, minimize pain associated with C-fibers, accommodation or adaptation of C-fibers, etc. A shorter pulse width (and phase width) reduces activation of C-fibers while a sufficient duty cycle may ensures activation of target tissue (e.g., K cells, L cells, etc.) for purposes of treating a metabolic disorder and/or a cardiac condition.

### Exemplary Methods

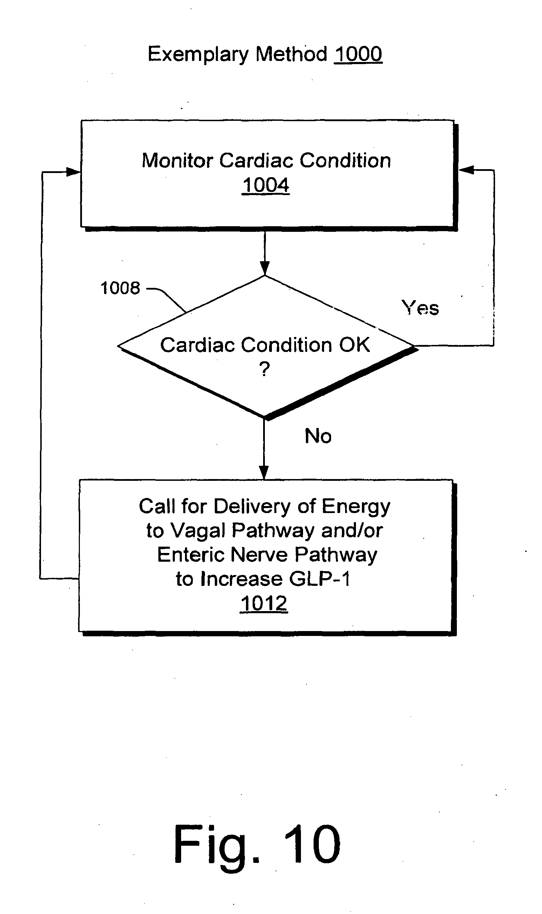

Fig. 10 shows an exemplary method 1000 for treating a cardiac condition. The method 1000 includes a monitor block 1004 to acquire information related to cardiac condition. The acquired information may be via one or more sensors (e.g., electrodes or other types of sensors) or the acquired information may be acquired via wireless transmission. For example, a patient may initiate transmission of information via a transmission device to indicate a cardiac condition. With respect to various scenarios of Fig. 1, the transmission device may be an external device that can transmit information to an implantable device.

The method 1000 uses the acquired information in a decision block 1008 that decides if the cardiac condition is OK. In an alternative method, the acquired information may be an alert that already indicates that cardiac condition is not OK. If the decision block 1008 decides that the cardiac condition is OK, then the method 1000 returns to the acquisition block 1004 or takes other appropriate action (e.g., according to a schedule, sensed information, transmitted information, etc.). However, if the decision block 1008 decides that the cardiac condition is not OK, then a call block 1012 calls for delivery of energy to a vagal pathway and/or enteric nerve pathway to increase GLP-1. As already mentioned, GLP-1 can have beneficial effects on cardiac condition and/or cardiac performance.

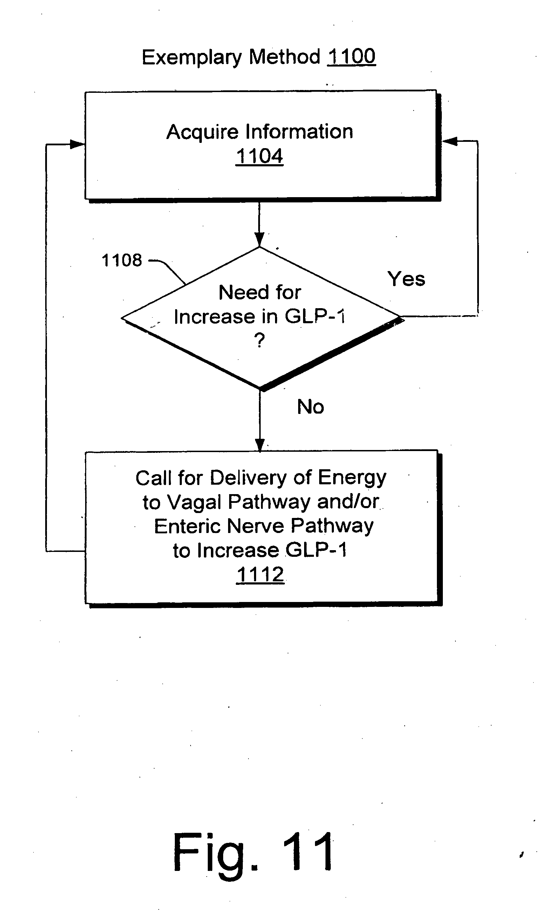

Fig. 11 shows an exemplary method 1100 for inducing release of GLP-1. The method 1100 includes an acquisition block 1104 to acquire information. The acquired information may be via one or more sensors (e.g., electrodes or other types of sensors) or the acquired information may be acquired via wireless transmission. For example, a patient may initiate transmission of information via a transmission device to indicate a need for release of GLP-1. With respect to various scenarios of Fig. 1, the transmission device may be an external device that can transmit information to an implantable device.

The method 1100 uses the acquired information in a decision block 1108 that decides if a need for an increase in GLP-1 exists. In an alternative method, the acquired information may be an alert that already indicates that a need for an increase in GLP-1 exists. If the decision block 1108 decides that a need does not exist, then the method 1100 returns to the acquisition block 1104 or takes other appropriate action (e.g., according to a schedule, sensed information, transmitted information, etc.). However, if the decision block 1108 decides that a need exists for an increase in GLP-1, then a call block 1112 calls for delivery of energy to a vagal pathway and/or enteric nerve pathway to increase GLP-1. As already mentioned, GLP-1 can have beneficial effects on cardiac condition and/or cardiac performance. GLP-1 also has beneficial effects on diseases such as type 2 diabetes. An exemplary method optionally treats cardiac condition and treats a metabolic condition such as type 2 diabetes.

The methods 1000 and 1100 may be suitable implemented using various scenarios 102, 104, 106 and 108 of Fig. 1. The methods 1000 and 1100 may use one or more electrodes positioned approximately as shown in Figs. 7 and 8. The methods 1000 and 1100 may be implemented in the form of processor-executable instructions, for example, stored on one or more computer-readable media. An exemplary implantable device may implement the call blocks 1012 and 1112 in any of a variety of manners. For example, a patient may initiate a call block via an external device (transmission device, magnet, etc.), a condition may initiate a call block, etc.

As already mentioned with respect to Table 2, a method may include calling for coordinated delivery of energy to the duodenum and to the distal ileum (e.g., simultaneous, alternating, according to a schedule, etc.). Coordinated delivery may be tailored to a specific individual based on information acquired during a clinical examination, acquired via feedback (e.g., sensing or patient input), etc., and hence operate according to closed-loop or open-loop control. Coordinated delivery of energy can optionally occur according to a schedule in a manner that aims to cause secretion of GLP-1 at particular times of the day (e.g., as associated with patient activity, biological cycles, feeding, etc.). For example, an exemplary device can include a timer that triggers events according to a programmed schedule. A care provider may program such a device via an implantable device programmer. A patient may be signaled by an implantable device or by an external device to coordinate patient behavior with programmed action of the implantable device.

While various methods pertain to cardiac therapies, an exemplary method optionally treats a metabolic disorder in addition to treating a cardiac condition. A method that treats a metabolic disorder may include calling for delivery of energy to a portion of the intestine to cause secretion of GIP and calling for delivery of energy to a more distal portion of the intestine to cause secretion of GLP-1. In such a method the portion of the intestine may be a portion of the duodenum or jejunum and the more distal portion of the intestine may be a portion of the ileum. Such a method may call for delivery of energy in response to an event or receipt of information. For example, calling for delivery of energy to the portion to cause secretion of GIP may occur in response to detecting digestion of food (e.g., food intake) and/or calling for delivery of energy to the more distal portion to cause secretion of GLP-1 may occur in response to detecting digestion of food (e.g., food intake). A method may call for delivery of energy in response to detecting a high in vivo concentration of glucose (e.g., a gastrointestinal concentration or a blood concentration).

Various exemplary methods may include vagal activation. For example, an exemplary method may include calling for delivery of energy to a portion of the duodenum to activate vagal afferents where the vagal afferents cause secretion of GLP-1. An exemplary method may also activate local, intestinal nerves (see, e.g., mechanism described with respect to Fig. 5). Various exemplary methods may include vagal activation and other activation. For example, a method for treating a cardiac condition may include calling for delivery of energy to a portion of the duodenum to activate vagal afferents and calling for delivery of energy to a more distal portion of the intestine to cause secretion of GLP-1. In such a method, the vagal afferents cause secretion of GLP-1.

As already mentioned, various methods may be implemented using one or more implantable devices. For example, an exemplary implantable device may include a processor and one or more computer-readable media with processor executable instructions to call for delivery of energy to the duodenum to activate K cells using an implantable device and to call for delivery of energy to the distal ileum to activate L cells using an implantable device where delivery of such energy causes, directly or indirectly, secretion of GLP-1.

Although exemplary mechanisms (e.g., implemented as or in methods, devices, systems, software, etc.) have been described in language specific to structural features and/or methodological acts, it is to be understood that the subject matter defined in the appended claims is not necessarily limited to the specific features or acts described.

## Claims

1. An implantable device (110, 112, 114, 118) for diminishing long-term myocardial damage causes by an infarct or ischemia, the device comprising:
detection circuitry adapted to detect an infarct or ischemia, **characterised by**:
stimulation circuitry adapted to call, in response to the detection circuitry detecting an infarct or ischemia, for delivery of electrical stimulation to a vagal pathway and/or enteric nerve pathway to activate L cells in the distal ileum wherein activation of L cells causes release of GLP-1 and wherein release of GLP-1 diminishes long-term myocardial damage caused by an infarct or ischemia.

2. The device (110, 112, 114, 118) of claim 1 wherein the stimulation circuitry is adapted to call for delivery of energy to the duodenum to activate K cells in the proximal duodenum wherein activation of K cells causes release of GIP.

3. The device (110, 112, 114, 118) of claim 2 wherein the stimulation circuitry is adapted to call for coordinated delivery of energy to the duodenum and to the distal ileum.

4. The device (110, 112, 114, 118) of claim 1 wherein the detection circuitry analyzes an electrogram to detect an infarct or ischemia.

5. The device (110.-112, 114, 118) of claim 4 wherein the detection circuitry acquires at least one temperature or at least one temperature differential.

## Patentansprüche

1. Implantierbare Vorrichtung (110, 112, 114, 118) zum Mindern von durch einen Infarkt oder eine Ischämie verursachtem Langzeit-Herzmuskelschaden, wobei die Vorrichtung aufweist.
eine Erkennungsschaltung, die dazu ausgelegt ist, einen Infarkt oder Ischämie zu erkennen,
**gekennzeichnet durch**:
eine Stimulationsschaltung, die dazu ausgelegt ist, im Ansprechen darauf, dass die Erkennungsschaltung einen Infarkt oder eine Ischämie erkennt, das Zuführen elektrischer Stimulation für einen vagalen Pfad und/oder einen enteralen Nervenpfad hervorzurufen, um L Zellen im distalen Ileum zu aktivieren, wobei das Aktivieren von L Zellen das Freisetzen von GLP-1 verursacht, und wobei das Freisetzen von GLP-1 den **durch** einen Infarkt oder eine Ischämie verursachten Langzeit-Herzmuskelschaden mindert.

2. Vorrichtung (110, 112, 114, 118) nach Anspruch 1, wobei die Stimulationsschaltung dazu ausgelegt ist, die Zufuhr von Energie an das Duodenum hervorzurufen, um K Zellen im proximalen Duodenum zu aktivieren, wobei das Aktivieren von K Zellen das Freisetzen von GIP verursacht.

3. Vorrichtung (110, 112, 114, 118) nach Anspruch 2, wobei die Stimulationsschaltung dazu ausgelegt ist, die koordinierte Zufuhr von Energie an das Duodenum und an das distale Ileum hervorzurufen.

4. Vorrichtung (110, 112, 114, 118) nach Anspruch 1, wobei die Erkennungsschaltung ein Elektrogramm analysiert, um einen Infarkt oder eine Ischämie zu erkennen.

5. Vorrichtung (110, 112, 114, 118) nach Anspruch 4, wobei die Erkennungsschaltung wenigstens eine Temperatur oder wenigstens ein Temperaturdifferential aufnimmt.

## Revendications

1. Un appareil implantable (110, 112, 114, 118) visant à diminuer les lésions à long terme du myocarde provoquées par un infarctus ou une ischémie, l'appareil comprenant :
un circuit de détection adapté à la détection d'un infarctus ou d'une ischémie,
**caractérisé par** :
un circuit de stimulation adapté de façon à provoquer, en réponse à la détection par le circuit d'un infarctus ou d'une ischémie, la génération d'une stimulation par voie d'un nerf vague et/ou d'un nerf entérique afin d'activer les cellules L dans l'iléon distal où l'activation des cellules L provoque la libération de GLP-1 et où la libération de GLP-1 diminue les lésions à long terme du myocarde provoquées par un infarctus ou une ischémie.

2. L'appareil (110, 112, 114, 118) de la revendication 1, où le circuit de stimulation est adapté de façon à susciter la fourniture d'énergie au duodénum, afin d'activer les cellules K dans le duodénum proximal où l'activation des cellules K provoque la libération de GIP.

3. L'appareil (110, 112, 114, 118) de la revendication 1, où le circuit de stimulation est adapté de façon à susciter la fourniture coordonnée d'énergie au duodénum et à l'iléon distal.

4. L'appareil (110, 112, 114, 118) de la revendication 1, où le circuit de détection analyse un électrogramme afin de détecter un infarctus ou une ischémie.

5. L'appareil (110, 112, 114, 118) de la revendication 4 où le circuit de détection acquiert au moins une température ou au moins un différentiel de température.
